(19) Europäisches Patentamt
European Patent Office
Office européen des brevets

(11) Veröffentlichungsnummer: **0 031 086**
**A1**

(12) # EUROPÄISCHE PATENTANMELDUNG

(21) Anmeldenummer: **80107805.6**

(22) Anmeldetag: **11.12.80**

(51) Int. Cl.³: **C 07 D 233/90**
A 01 N 43/50

(30) Priorität: **19.12.79 DE 2951203**

(43) Veröffentlichungstag der Anmeldung:
**01.07.81 Patentblatt 81/26**

(84) Benannte Vertragsstaaten:
**BE CH DE FR GB IT LI NL**

(71) Anmelder: **BAYER AG**
**Zentralbereich Patente, Marken und Lizenzen**
**D-5090 Leverkusen 1, Bayerwerk(DE)**

(72) Erfinder: **Beck, Gunther, Dr.**
**Am Mittelberg 19**
**D-5090 Leverkusen(DE)**

(72) Erfinder: **Heitzer, Helmut, Dr.**
**Höhenstrasse 84**
**D-5090 Leverkusen(DE)**

(72) Erfinder: **Eue, Ludwig, Dr.**
**Paul-Klee-Strasse 36**
**D-5090 Leverkusen(DE)**

(72) Erfinder: **Schmidt, Robert Rudolf, Dr.**
**Hahnenweg 5**
**D-5000 Koeln(DE)**

(54) **Halogenierte Imidazol-carbonsäure-Derivate, Verfahren zu ihrer Herstellung sowie ihre Verwendung als Herbizide.**

(57) Neue halogenierte Imidazol-carbonsäure-Derivate der Formel

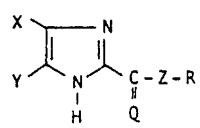

(I)

in welcher
X für Chlor, Brom oder Jod steht,
Y für Wasserstoff, Chlor, Brom oder Jod steht, wobei jedoch X und Y nicht gleichzeitig für Chlor stehen,
Z für Sauerstoff oder für die NH-Gruppe steht,
R für Wasserstoff oder Alkyl steht und
Q für Sauerstoff oder Schwefel steht oder der Rest der Formel $-\underset{\underset{Q}{\|}}{C}-Z-R$ für -C≡N steht,

mehrere Verfahren zur Herstellung dieser Stoffe und deren Verwendung als Herbizide.

EP 0 031 086 A1

BAYER AKTIENGESELLSCHAFT 5090 Leverkusen, Bayerwerk

Zentralbereich
Patente, Marken und Lizenzen Dü/Kü-c
Ib

## Halogenierte Imidazol-carbonsäure-Derivate, Verfahren zu ihrer Herstellung sowie ihre Verwendung als Herbizide

Die vorliegende Erfindung betrifft neue halogenierte Imidazol-carbonsäure-Derivate, mehrere Verfahren zu ihrer Herstellung sowie ihre Verwendung als Herbizide.

Es ist bereits bekannt geworden, daß bestimmte 4,5-Dichlorimidazol-2-carbonsäure-Derivate herbizide Eigenschaften besitzen (vgl. DE-OS 2 610 527). So kann zum Beispiel das 4,5-Dichlorimidazol-2-carbonsäure-ethylamid zur Bekämpfung von Unkraut eingesetzt werden. Dieser Stoff ist jedoch nicht immer ausreichend wirksam und in seiner Selektivität nicht immer ganz befriedigend.

Es wurden nun neue halogenierte Imidazolcarbonsäure-Derivate der Formel

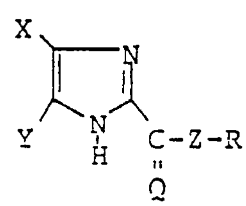

(I)

in welcher

X für Chlor, Brom oder Jod steht,

Y für Wasserstoff, Chlor, Brom oder Jod steht, wobei jedoch X und Y nicht gleichzeitig für Chlor stehen,

Z für Sauerstoff oder für die NH-Gruppe steht,

R für Wasserstoff oder Alkyl steht und

Q für Sauerstoff oder Schwefel steht oder der Rest der Formel $-\underset{\underset{Q}{\|}}{C}-Z-R$ für $-C\equiv N$ steht,

gefunden.

Weiterhin wurde gefunden, daß man halogenierte Imidazol-carbonsäure-Derivate der Formel (I) erhält, wenn mann

a) Imidazol-2-carbonsäure-Derivate der Formel

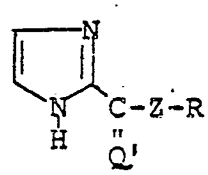

(II)

in welcher

Z und R die oben angegebene Bedeutung haben und

Q' für Sauerstoff steht
oder der Rest der Formel $-\underset{\underset{Q'}{\|}}{C}-Z-R$ für -CN steht,

mit maximal der zur Monohalogenierung stöchiometrisch erforderlichen Menge an Halogenierungsmittel in Gegenwart eines unter den Reaktionsbedingungen inerten Verdünnungsmittels umsetzt, oder

b) Imidazol-2-carbonsäure-Derivate der Formel

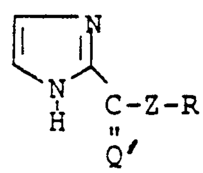

(II)

in welcher

Z, R und Q' die oben angegebene Bedeutung haben,

mit mindestens der zur Dihalogenierung stöchiometrisch erforderlichen Menge an Halogenierungsmittel in Gegenwart eines unter den Reaktionsbedingungen inerten Verdünnungsmittels sowie gegebenenfalls in Gegenwart eines säurebindenden Mittels umsetzt, oder

c) monohalogenierte Imidazol-2-carbonsäure-Derivate der Formel

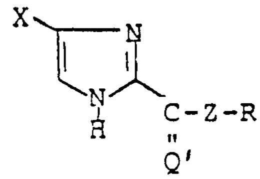

(III)

in welcher

X, Z, R und Q' die oben angegebene Bedeutung haben,

mit der stöchiometrisch erforderlichen Menge oder mit einem Überschuß an Halogenierungsmittel in Gegenwart eines unter den Reaktionsbedingungen inerten Verdünnungsmittels sowie gegebenenfalls in Gegenwart eines Säurefängers umsetzt,

oder

d) halogenierte Imidazol-carbonsäure-derivate der Formel

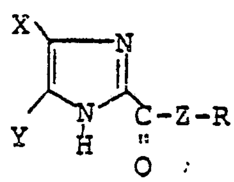

(IV)

in welcher

X, Y, Z und R die oben angegebene Bedeutung haben,

mit mindestens der stöchiometrisch erforderlichen Menge an Tetraphosphor-dekasulfid ("Phosphorpentasulfid") in Gegenwart eines unter den Reaktionsbedingungen inerten Verdünnungsmittels umsetzt.

Schließlich wurde gefunden, daß die neuen halogenierten Imidazol-carbonsäure-Derivate der Formel (I) starke herbizide, insbesondere auch selektiv-herbizide Eigenschaften besitzen.

Überraschenderweise zeigen die erfindungsgemäßen halogenierten Imidazol-carbonsäure-Derivate der Formel (I) bei sehr guter Unkrautwirkung insbesondere bessere Möglichkeiten zum Einsatz als selektive Unkrautbekämpfungsmittel in wichtigen Kulturen als das aus dem Stand der Technik bekannte 4,5-Dichlorimidazol-2-carbonsäure-ethylamid, welches ein hochaktiver Wirkstoff gleicher Wirkungsart ist. Die erfindungsgemäßen Stoffe stellen somit eine wertvolle Bereicherung der herbiziden Mittel zur selektiven Unkrautbekämpfung dar.

Die erfindungsgemäßen halogenierten Imidazol-carbonsäure-Derivate sind durch die Formel (I) allgemein definiert. In dieser Formel steht R vorzugsweise für Wasserstoff oder geradkettiges oder verzweigtes Alkyl mit 1 bis 18 Kohlenstoffatomen.

Besonders bevorzugt sind diejenigen Verbindungen der Formel (I), in denen X und Z die oben angegebene Bedeutung haben, Y für Wasserstoff steht, Q für Sauerstoff steht und R für Wasserstoff oder geradkettiges oder verzweigtes Alkyl mit 1 bis 18 Kohlenstoffatomen steht.

Weiterhin sind diejenigen Verbindungen der Formel (I) besonders bevorzugt, in denen X die oben angegebene Bedeutung hat, Y für Chlor, Brom oder Jod steht, wobei jedoch X und Y nicht gleichzeitig für Chlor stehen, Q für Sauerstoff steht oder der Rest $-\underset{\underset{Q}{\|}}{C}-Z-R$ für -CN steht, Z für Sauerstoff oder NH steht und R für Wasserstoff oder geradkettiges oder verzweigtes Alkyl mit 1 bis 18 Kohlenstoffatomen steht.

Außerdem sind diejenigen Verbindungen der Formel (I) besonders bevorzugt, in denen X die oben angegebene Bedeutung hat, Y für Wasserstoff, Chlor, Brom oder Jod steht, wobei jedoch X und Y nicht gleichzeitig für Chlor stehen, Z für die NH-Gruppe steht, Q für Schwefel steht und R für Wasserstoff oder geradkettiges oder verzweigtes Alkyl mit 1 bis 18 Kohlenstoffatomen steht.

Verwendet man bei dem erfindungsgemäßen Verfahren (a) Imidazol-2-carbonsäure-tert.-butylamid und Sulfurylchlorid in solchen Mengen, daß das Sulfurylchlorid in weniger als der zur Monochlorierung stöchiometrisch erforderlichen Menge vorhanden ist, so kann der Verlauf der Umsetzung durch das folgende Formelschema wiedergegeben werden:

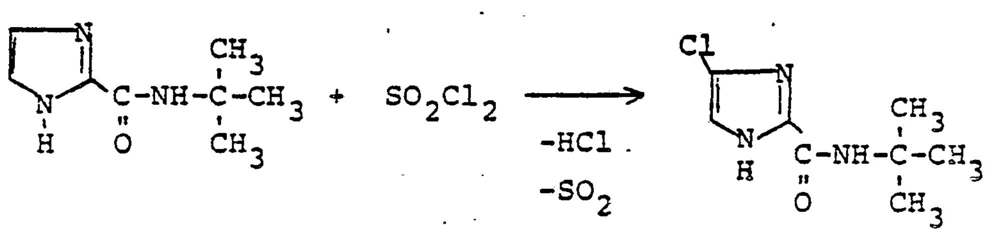

Verwendet man bei dem erfindungsgemäßen Verfahren (b) Imidazol-2-carbonsäure-tert.-butylamid und Brom in solchen Mengen, daß das Brom mindestens in der zur Dibromierung stöchiometrisch erforderlichen Menge vorhanden ist, so kann der Verlauf der Umsetzung durch das folgende Formelschema wiedergegeben werden:

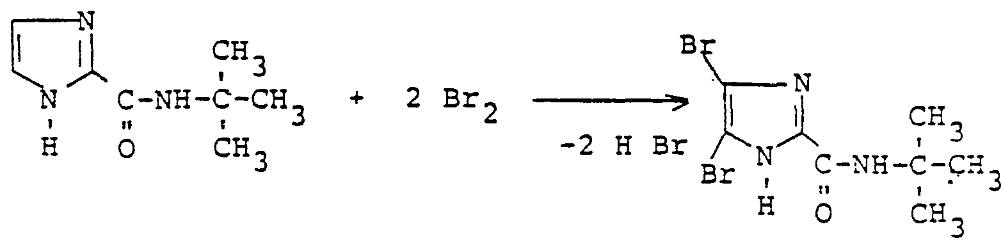


Verwendet man bei dem erfindungsgemäßen Verfahren (c) 4-Brom-imidazol-2-carbonsäure-tert.-butylamid und Sulfurylchlorid als Ausgangsstoffe, so kann der Verlauf der Umsetzung durch das folgende Formelschema wiedergegeben werden:

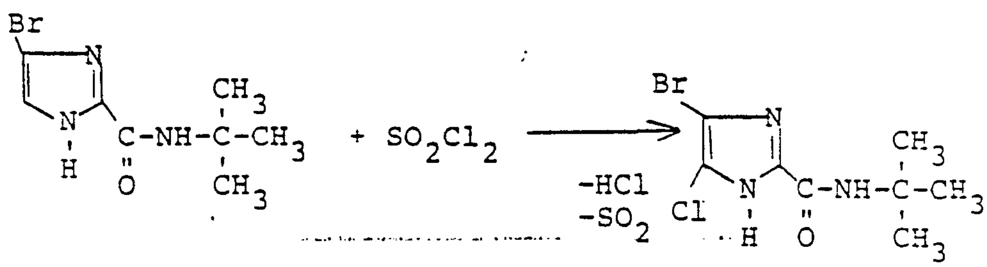


Verwendet man bei dem erfindungsggemäßen Verfahren (d) 4,5-Dibrom-imidazol-2-carbonsäure-tert.-butylamid und Tetraphosphor-dekasulfid ("Phosphorpentasulfid") als Ausgangsstoffe, so kann der Verlauf der Umsetzung durch das folgende Formelschema wiedergegeben werden:

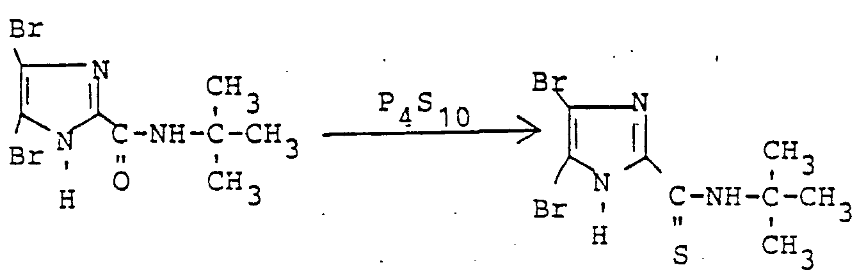

Die bei dem erfindungsgemäßen Verfahren (a) als Ausgangsstoffe benötigten Imidazol-2-carbonsäure-Derivate sind durch die Formel (II) allgemein definiert. In dieser Formel steht Z für Sauerstoff oder die NH-Gruppe, R steht für Wasserstoff oder Alkyl, vorzugsweise mit 1 bis 18 Kohlenstoffatomen, und Q' steht für Sauerstoff oder der Rest $-\underset{\underset{Q'}{\|}}{C}-Z-R$ steht für die -CN-Gruppe.

Die Imidazol-2-carbonsäure-Derivate der Formel (II) sind teilweise bekannt, (vgl. Acta Chem. Scand. <u>21</u>, 279 (1967); Recl. Trav. Chim. Pays-Bas <u>1971</u> (90), Seite 207 und J. Org. Chem. <u>1977</u> (42), Seite 3925).

Die bisher noch nicht beschriebenen Imidazol-2-carbonsäure-Derivate der Formel (II) lassen sich in einfacher Weise nach verschiedenen Methoden herstellen. So erhält man Verbindungen der Formel (II), indem man

e) Imidazol mit Isocyanaten der Formel

$$R' - NCO \qquad (V)$$

in welcher

R' für Alkyl steht

in Gegenwart eines Verdünnungsmittels, wie zum

Beispiel Nitrobenzol, bei Temperaturen zwischen 150°C und 250°C umsetzt,

bzw.

f) das dimere Keten der Formel

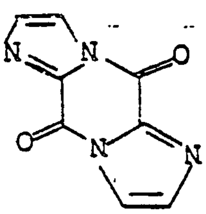

(VI)

mit Aminen oder Alkoholen der Formel

H - Z - R' (VII)

in welcher

Z und R' die oben angegebene Bedeutung haben,

gegebenenfalls in Gegenwart eines Verdünnungsmittels sowie gegebenenfalls in Gegenwart eines Katalysators bei Temperaturen zwischen -50°C und +200°C umsetzt.

Die Isocyanate der Formel (V) sind ebenso wie die Amine und Alkohole der Formel (VII) bereits bekannt. Das dimere Keten der Formel (VI) hingegen ist bisher

noch nicht beschrieben worden. Es läßt sich herstellen, indem man Imidazol-2-carbonsäure mit einem Überschuß an Thionylchlorid gegebenenfalls in Gegenwart von Dimethylformamid als Katalysator unter Rückfluß erhitzt.

Als Verdünnungsmittel kommen bei der Umsetzung nach Verfahren (f) sowohl die als Reaktionskomponenten verwendeten Alkohole bzw. Amine der Formel (VII) selbst, -sofern sie in flüssigem Zustand vorliegen oder bei niedrigen Temperaturen schmelzen-, als auch inerte organische Solventien, wie Methylenchlorid oder Chloroform, in Frage. Insbesondere bei der Umsetzung von Aminen der Formel (VII) mit dem dimeren Keten der Formel (VI) können niedere Alkohole ($C_1$-$C_4$), wie Methanol oder Isopropanol, oder auch Wasser als Verdünnungsmittel fungieren.

Als Katalysatoren können bei dem Verfahren (f) starke anorganische Basen, wie Natriumhydroxid oder Kaliumhydroxid, oder Amine, wie Triethylamin oder Triethylendiamin, verwendet werden. Der Einsatz von Katalysatoren empfiehlt sich insbesondere dann, wenn es sich bei der Reaktionskomponente der Formel (VII) um einen Alkohol handelt.

Die Reaktionstemperaturen können bei dem Verfahren (f) innerhalb eines größeren Bereiches variiert werden. Im allgemeinen arbeitet man bei Temperaturen zwischen -50°C und +200°C, vorzugsweise zwischen 0°C und 100°C.

Bei der Durchführung des Verfahrens (f) setzt man auf 1 Mol an dimerem Keten der Formel (VI) 2 Mol oder auch einen größeren Überschuß an Amin bzw. Alkohol der Formel (VII) sowie gegebenenfalls 0,01 bis 1, vorzugsweise 0,1 bis 0,5 Mol an Katalysator ein. Die Isolierung der entstehenden Imidazol-2-carbonsäure-Derivate der Formel (II) erfolgt nach üblichen Methoden. Im allgemeinen verfährt man in der Weise, daß man nach beendeter Umsetzung das Lösungsmittel und/oder die im Überschuß vorhandene Komponente (VII) abdestilliert und den verbleibenden Rückstand gegebenenfalls umkristallisiert oder unter vermindertem Druck sublimiert.

Als Halogenierungsmittel kommen bei dem erfindungsgemäßen Verfahren (a) die Halogene Chlor, Brom und Jod in Frage. Zur Chlorierung können auch Chlor abgebende Substanzen, wie Sulfurylchlorid, verwendet werden.

Als Verdünnungsmittel kommen bei dem erfindungsgemäßen Verfahren (a) alle unter den Reaktionsbedingungen inerten Verdünnungsmittel in Betracht. Hierzu gehören vorzugsweise Methylenchlorid und Chloroform.

Die Reaktionstemperaturen können bei dem erfindungsgemäßen Verfahren (a) innerhalb eines größeren Bereiches variiert werden. Im allgemeinen arbeitet man bei Temperaturen zwischen -50°C und +100°C, vorzugsweise zwischen -20°C und +50°C.

Bei der Durchführung des erfindungsgemäßen Verfahrens (a) setzt man auf 1 Mol an Imidazol-2-carbonsäure-Derivat der Formel (II) 0,1 bis zu maximal 1 Mol, vorzugsweise 0,3 bis 0,5 Mol an Halogen bzw. eine entsprechende Menge an einer Chlor freisetzenden Substanz ein. Im einzelnen verfährt man in der Weise, daß man das Imidazol-2-carbonsäure-Derivat der Formel (II) in einem Lösungsmittel vorlegt und das Halogen in elementarer Form oder in Lösung, bzw. die Chlor abgebende Substanz gegebenenfalls unter Kühlung so zugibt, daß die gewählte Reaktionstemperatur eingehalten wird. Die Aufarbeitung erfolgt im allgemeinen dadurch, daß man zunächst das Lösungsmitttel abzieht und den Rückstand zur Abtrennung von mit enstandenem dihalogenierten Produkt bei Raumtemperatur mit Salzsäure im Verhältnis 1:1 versetzt. Dabei verbleibt das dihalogenierte Produkt in festem Zustand, während das monohalogenierte Produkt sowie das nicht halogenierte Ausgangsprodukt in Lösung gehen. Das Festprodukt wird abfiltriert. Das salzsaure Filtrat wird zur Abtrennung von noch enthaltenem nicht halogenierten Ausgangsprodukt so mit wässriger Alkalilauge versetzt, daß der pH-Wert der Lösung etwas ansteigt. Hierbei bleibt das Ausgangsprodukt der Formel (II) in Lösung, während das gewünschte monohalogenierte Produkt ausfällt. Lezteres wird durch Filtration abgetrennt.

Bei dem erfindungsgemäßen Verfahren (b) kommen als Ausgangsstoffe alle diejenigen Imidazol-2-carbonsäure-Derivate der Formel (II) in Betracht, die auch im Falle des erfindungsgemäßen Verfahrens (a) als Ausgangssubstanzen fungieren.

Als Halogenierungsmittel können bei dem erfindungsgemäßen Verfahren (b) die Halogene Brom und Jod verwendet werden.

Als Säurebindemittel kommen bei dem erfindungsgemäßen Verfahren (b) vorzugsweise Alkali- oder Erdalkalihydroxide, -carbonate oder -hydrogencarbonate, wie zum Beispiel Natriumhydroxid oder Natriumhydrogencarbonat, in Frage.

Als Verdünnungsmittel kommen bei dem erfindungsgemäßen Verfahren (b) alle unter den Reaktionsbedindungen inerten Lösungsmittel in Betracht. Hierzu gehören vorzugsweise Methylenchlorid und Chloroform. Es kann jedoch auch in wässrigem Medium gegebenenfalls in Gegenwart eines Säurefängers gearbeitet werden.

Die Reaktionstemperaturen können bei dem erfindungsgemäßen Verfahren (b) innerhalb eines größeren Bereiches variiert werden. Im allgemeinen arbeitet man bei Temperaturen zwischen -50°C und +150°C, vorzugsweise zwischen -20°C und +100°C. Dient Wasser als Verdünnungsmittel, so arbeitet man zweckmäßigerweise zwischen 0°C und +50°C.

Bei der Durchführung des erfindungsgemäßen Verfahrens (b) setzt man auf 1 Mol an Imidazol-2-carbonsäure-Derivat der Formel (II) 2 Mol oder auch einen größeren Überschuß, vorzugsweise 2,5 Mol an Halogen sowie bei Verwendung von Wasser als Verdünnungsmittel 2 oder mehr Äquivalente eines Säurebinders ein. Im einzelnen verfährt man so, daß man das Imidazol-2-carbonsäure-Derivat der Formel

(II) in einem Lösungsmittel vorlegt, dann das Halogenierungsmittel, gegebenenfalls unter Kühlung, in dem Maße zudosiert, daß die gewählte Reaktionstemperatur eingehalten wird. Zur Vervollständigung der Reaktion kann das Reaktionsgemisch nach beendeter Zugabe des Halogenierungsmittels noch weitere 1 bis 5 Stunden auf der gewählten Temperatur gehalten werden. Im Falle der Verwendung von Wasser als Verdünnungsmittel kann der Säurefänger entweder mit dem Imidazol-2-carbonsäure-Derivat der Formel (II) vorgelegt werden oder aber nach der Zugabe des Halogenierungsmittels zugefügt werden. Auch die gleichzeitige Zugabe von Säurefänger und Halogenierungsmittel ist möglich. In einer weiteren Variante kann auch das Halogenierungsmittel vorgelegt werden und das Imidazol-2-carbonsäure-Derivat der Formel (II) in Lösung hinzugegeben werden. Die Isolierung der Reaktionsprodukte erfolgt nach üblichen Methoden. Im allgemeinen verfährt man so, daß man bei Verwendung von inerten organischen Solventien als Verdünnungsmittel letztere nach beendeter Umsetzung abzieht und den Rückstand gegebenenfalls reinigt, indem man das Rohprodukt mit wässriger Salzsäure im Verhältnis 1:1 bei 20°C behandelt. Dabei gehen monohalogenierte Verbindungen und nicht halogeniertes Ausgangsprodukt in Lösung, während das dihalogenierte Produkt in fester Form verbleibt und abfiltriert werden kann. Bei Jodierungen werden eventuell anfallende Hydrojodide zweckmäßigerweise dadurch in die entsprechenden Jodwasserstofffreien Produkte überführt, daß man sie bei Raumtemperatur in wässriger 10 %-iger Natronlauge löst, gegebenenfalls vorhandene geringfügige Verunreinigungen abfiltriert und anschließend das Produkt durch Ansäuern mit Salzsäure wieder ausfällt und abfiltriert.

Dient Wasser bei dem erfindungsgemäßen Verfahren (b) als Reaktionsmedium, so fällt das in Wasser praktisch unlösliche Produkt unmittelbar in fester Form an, so daß es abfiltriert werden kann.

Die für das erfindungsgemäße Verfahren (c) als Ausgangsprodukte benötigten monohalogenierten Imidazol-2-carbonsäure-Derivate sind durch die Formel (III) allgemein definiert. In dieser Formel haben X, Z und Q' diejenigen Bedeutungen, die bereits im Zusammenhang mit der Beschreibung der erfindungsgemäßen Verbindungen der Formel (I) bzw. der Verbindungen der Formel (II) genannt wurden. R steht vorzugsweise für diejenigen Reste, die im Zusammenhang mit der Beschreibung der erfindungsgemäßen Stoffe der Formel (I) vorzugsweise für R genannt wurden.

Die monohalogenierten Imidazol-2-carbonsäure-Derivate der Formel (III) sind bisher noch nicht bekannt. Sie lassen sich jedoch nach dem erfindungsgemäßen Verfahren (a) in einfacher Weise herstelllen.

Als Halogenierungsmittel kommen bei dem erfindungsgemäßen Verfahren (c) außer den Halogenen Chlor, Brom und Jod auch Chlor abgebende Substanzen, wie Sulfurylchlorid, in Frage.

Als Säurebindemittel kommen alle diejenigen Stoffe in Betracht, die bereits im Zusammehang mit dem erfindungsgemäßen Verfahren (b) als Säurefänger erwähnt wurden.

Als Verdünnungsmittel kommen bei dem erfindungsgemäßen Verfahren (c) wiederum alle unter den Reaktionsbedingungen inerten Lösungsmittel in Betracht. Hierzu gehören vorzugsweise Methylenchlorid und Chloroform. Im Falle von Bromierungen und Jodierungen kann auch in wässrigem Medium, gegebenenfalls in Gegenwart von Säurefängern, gearbeitet werden.

Die Reaktionstemperaturen können bei dem erfingungsgemäßen Verfahren (c) in gleicher Weise variiert werden wie bei dem erfindungsgemäßen Verfahren (b).

Bei dem erfindungsgemäßen Verfahren (c) erfolgen die Durchführung der Umsetzung und die Aufarbeitung des Reaktionsgemisches analog zu dem erfindungsgemäßen Verfahren (b).

Die für das erfindungsgemäße Verfahren (d) als Ausgangsstoffe benötigten halogenierten Imidazol-carbonsäure-Derivate sind durch die Formel (IV) allgemein definiert. In dieser Formel haben X, Y und Z diejenigen Bedeutungen, die bereits im Zusammenhang mit der Beschreibung der erfindungsgemäßen Stoffe der Formel (I) für diese Reste genannt wurden. R steht vorzugsweise für diejenigen Reste, die im Zusammehang mit der Beschreibung der erfindungsgemäßen Stoffe der Formel (I) vorzugsweise für R erwähnt wurden.

Die halogenierten Imidazol-carbonsäure-Derivate der Formel (IV) sind bisher noch nicht bekannt. Sie lassen sich jedoch nach den erfindungsgemäßen Verfahren (a) bis (c) in einfacher Weise herstellen.

Die Umsetzung nach dem erfindungsgemäßen Verfahren (d) wird in Gegenwart eines unter den Reaktionsbedindungen inerten Verdünnungsmittels durchgeführt. Als derartige Verdünnungsmittel kommen vorzugsweise in Frage inerte Kohlenwasserstoffe, insbesondere aromatische Kohlenwasserstoffe und chlorierte aromatische Kohlenwasserstoffe, wie Toluol, Xylol und Chlorbenzol, ferner Azaaromaten, wie Pyridin, sowie Dioxan.

Die Reaktonstemperaturen können bei dem erfindungsgemäßen Verfahren (d) innerhalb eines größeren Bereiches variiert werden. Im allgemeinen arbeitet man bei Temperaturen zwischen 50 und 250°C, vorzugsweise zwischen 100 und 180°C.

Bei der Durchführung des erfindungsgemäßen Verfahrens (d) setzt man halogenierte Imidazol-carbonsäure-Derivate der Formel (IV) mit der stöchiometrisch erforderlichen Menge an Tetraphosphor-dekasulfid oder auch mit einem größeren Überschuß davon um. In der Praxis werden die beiden Reaktionskomponenten meist in etwa gleichen Gewichtsteilen verwendet. Im einzelnen verfährt man bei der Durchführung des erfindungsgemäßen Verfahrens (d) so, daß man das halogenierte Imidazol-carbonsäure-Derivat der Formel (IV) in einem Lösungsmittel mit etwa der gleichen Menge an Tetraphosphor-dekasulfid bis zur Beendigung der Umsetzung (0,5 bis 10 Stunden) unter Rückfluß erhitzt. Die Aufarbeitung erfolgt nach üblichen Methoden. Im allgemeinen verfährt man in der Weise, daß man das Lösungsmittel abdestilliert und überschüssiges Tetraphosphor-dekasulfid abtrennt, indem man das Reaktionsgemisch mit verdünnten, wässrigen Alkalien, wie zum Beispiel wässriger Natriumhydrogencarbonat-Lösung, erhitzt. Dabei wird das Tetraphosphor-dekasulfid zerstört, während das gewünschte Produkt in Lösung geht. Nach dem Abfiltrieren von ungelösten Verunreinigungen wird das Filtrat angesäuert. Das dabei ausfallende Reaktionsprodukt wird anschließend abfiltriert.

Die erfindungsgemäßen Wirkstoffe beeinflussen das Pflanzenwachstum und können deshalb als Defoliants, Desiccants, Krautabtötungsmittel, Keimhemmungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

<u>Dikotyle Unkräter der Gattungen</u>: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dicotyle Kulturen der Gattungen: Gossypium, Clycine, Beta, Daucus, Phaseolus, Pisum, Solanum, Linum, Ipomoea, Vicia, Nicotiana, Lycopersicon, Arachis, Brassica, Lactuca, Cucumis, Cuburbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum Ischaemum, Sphenocela, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen z.B. Forst-, Ziergehölz-, Obst-, Wein-, Citrus-, Nuss-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Stoffe eignen sich insbesondere zur selektiven Unkrautbekämpfung in Mais, Baumwolle und Getreide.

Die Wirkstoffe können in die üblichen Formulierungen übergeführt werden, wie Lösungen, Emulsionen, Suspensionen, Pulver, Schäume, Granulate, Wirkstoff-imprägnierte Natur- und synthetische Stoffe, Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln, und/oder festen Trägerstoffen, gegebenenfalls unter Verwendung von oberflächenaktiven Mittel, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; als feste Trägerstoffe kommen in Frage:

z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyäthylen-Fettsäure-Ester, Polyoxyäthylen-Fettalkohol-Äther, z.B. Alkylarylpolyglykol-äther, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige und latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo -Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischung möglich ist. Auch eine Mischung mit anderen bekannten Wirkstoffen, wir Fungiziden, Insektiziden, Akariziden, Nematiziden, Schutzstoffen gegen Vogelfraß, Wuchsstoffen, Pflanzennährstoffen und Bodenstrukturverbesserungsmitteln ist möglich.

Die Wirkstoffe können als solche, in Form ihrer Formulierungen oder der daraus durch weiteres Verdünnen bereiteten Anwendungsformen, wie gebrauchsfertige Lösungen, Suspensionen, Emulsionen, Pulver und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Die Anwendung wird vorzugsweise vor dem Auflaufen der Pflanzen, also im pre-emergence-Verfahren, vorgenommen. Sie können auch vor der Saat in den Boden eingearbeitet werden.

Die aufgewandte Wirkstoffmenge kann in größeren Bereichen schwanken. Sie hängt im wesentlichen von der Art des gewünschten Effekts ab. Im allgemeinen liegen die

Aufwandmengen zwischen 0,05 und 10 kg Wirkstoff pro ha, vorzugsweise zwischen 0,1 und 5 kg/ha.

Die Herstellung der erfindungsgemäßen Stoffe geht aus den nachfolgenden Beispielen hervor.

Beispiel 1

a)

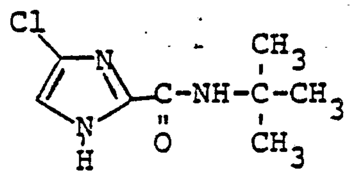

Zu einer Lösung von 87,8 g (0,52 Mol) Imidazol-2-carbonsäure-tert.-butylamid in 750 ml Methylenchlorid wurde unter Kühlung im Temperaturbereich von -10°C bis 0°C eine Mischung aus 28,1 g (0,21 Mol) Sulfurylchlorid und 250 ml Methylenchlorid getropft. Anschließend wurde etwa 1,5 Stunden bei Raumtemperatur nachgerührt. Danach wurde das Methylenchlorid im Vakuum abgezogen, der Rückstand mit 500 g wäßriger Salzsäure (1:1) verrührt, abgesaugt und das Filtrat mit Natronlauge auf einen pH-Wert von 1-2 abgestumpft. Der hierbei ausfallende Niederschlag wurde abgesaugt, mit Wasser nachgewaschen und getrocknet. Man erhielt so 10,8 g (entsprechend einer theoretischen Ausbeute von 25,4 %, bezogen auf eingesetztes Sulfurylchlorid) 4-Chlor-imidazol-2-carbonsäure-tert.-butylamid. Schmelzpunkt 155°C (aus Petrolether).

b) Herstellung des Ausgangsproduktes

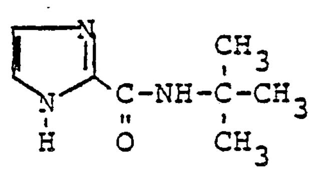

Zu einer Suspension von 188 g (1 Mol) des dimeren Ketens der Formel

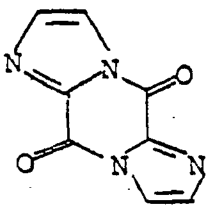

in 1,5 Liter Methylenchlorid wurden allmählich bei Raumtemperatur 153 g (2,1 Mol) tert.-Butylamin zugegeben, wobei die Temperatur des Reaktionsgemisches bis zum Siedepunkt (ca. 42°C) anstieg. Nach einstündigem Nachrühren unter Rückfluß wurde heiß von geringen Mengen an ungelöstem Produkt abfiltriert und das Filtrat im Vakuum zur Trockne eingeengt. Man erhielt so das Imidazol-2-carbonsäure-tert.-butylamid in praktisch quantitativer Ausbeute. Die Verbindung ist bei 100-120°C/0,01 Torr unzersetzt sublimierbar und aus Wasser in Säulen oder aus Petrolether (Kp. 40-80°C) in langen Nadeln kristallisierbar. Schmelzpunkt 134°C.

c) Herstellung des Vorproduktes

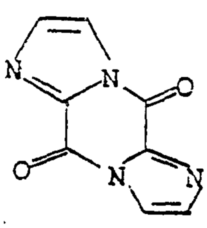

Variante α

11,2 g (0,1 Mol) Imidazol-2-carbonsäure wurden mit 100 ml Thionylchlorid 5 Stunden unter Rückfluß gerührt. Nach dem Erkalten wurde abgesaugt, mit etwas Petrolether gewaschen und getrocknet. Man erhielt so in fast quantitativer Ausbeute das 5H,10H-diimidazo/1,2-a:1',2'-d/pyrazin-5,10-dion in Form eines gelben Pulvers. Die

Substanz schmilzt noch nicht bei 290°C. Sie ist bei 200-250°C/0,01 Torr unzersetzt in gelben Kristallen sublimierbar.
IR (KBr): 3137, 1735, 1522, 1445, 1387, 1331, 1274, 1161, 1059, 1018, 810, 800, 748, 699, 651 $cm^{-1}$.

Variante ß

Man verfuhr wie unter ℒ) mit dem Unterschied, daß man 1 ml Dimethylformamid zusetzte. Die Umsetzung zur Verbindung der Formel

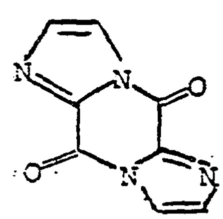

war, wie der Vergleich der IR-Spektren ergab, bereits nach etwa einer halben Stunde praktisch vollständig.

Beispiel 2

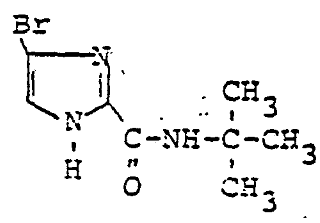

Zu einer Lösung von 39,8 g (0,238 Mol) Imidazol-2-carbonsäure-tert.-butylamid in 300 ml Methylenchlorid wurde unter Kühlung zwischen -10 und 0°C eine Mischung aus 8,32 g (0,052 Mol) Brom und 300 ml

Methylenchlorid innerhalb einer Stunde getropft. Nach zweistündigem Nachrühren bei Raumtemperatur wurde das Methylenchlorid im Vakuum abgezogen, der Rückstand mit 500 g wässriger Salzsäure (1:1) verrührt, abgesaugt und das Filtrat mit Natronlauge auf einen pH-Wert von 1-2 abgestumpft. Der hierbei ausfallende Niederschlag wurde abgesaugt, mit Wasser nachgewaschen und getrocknet. Man erhielt so 6,33 g (entsprechend einer theoretischen Ausbeute von 49,5 %, bezogen auf eingesetztes Brom) 4-Brom-imidazol-2-carbonsäure-tert.-butylamid. Schmelzpunkt 164°C (aus Petroläther).

Beispiel 3

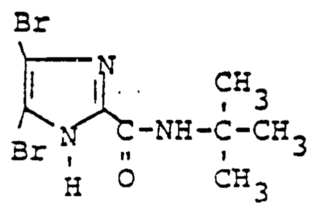

α) in Methylenchlorid

Zu einer Lösung von 16,7 g (0,1 Mol) Imidazol-2-carbonsäure-tert.-butylamid in 200 ml Methylenchlorid wurde bei etwa 20°C eine Mischung aus 35,2 g (0,22 Mol) Brom und 50 ml Methylenchlorid getropft. Danach wurde 3 Stunden unter Rückfluß gerührt. Anschließend wurde das Methylenchlorid und das überschüssige Brom im Vakuum abgezogen und der Rückstand mit Wasser (ca.

500 ml) verrührt. Die saure Suspension wurde mit Natronlauge neutralisiert, der Niederschlag abgesaugt, mit Wasser gewaschen und getrocknet. Man erhielt so 22,2 g (68,3 % der Theorie) 4,5-Dibromimidazol-2-carbonsäure-tert.-butylamid. Schmelzpunkt 258°C (aus Acetonitril).

β) in Wasser

Eine Lösung von 6,72 g (0,08 Mol) Natriumhydrogencarbonat in 100 ml Wasser wurde zunächst mit 6,7 g (0,04 Mol) Imidazol-2-carbonsäure-tert.-butylamid versetzt. Anschließend wurden bei 15 bis 20°C 14,1 g (0,088 Mol) Brom zugetropft und 2 Stunden bei Raumtemperatur nachgerührt. Danach wurde der Niederschlag abgesaugt, mit Wasser nachgewaschen und getrocknet. Man erhielt so 10,9 g (83,8 % der Theorie) 4,5-Dibromimidazol-2-carbonsäure-tert.-butylamid, identisch mit der unter α) erhaltenen Verbindung.

Beispiel 4

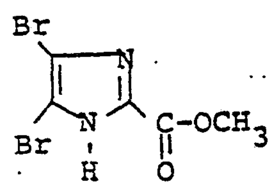

Eine Lösung von 16,8 g (0,2 Mol) Natriumhydrogencarbonat in 250 ml Wasser wurde zunächst mit 12,6 g (0,1 Mol) Imidazol-2-carbonsäuremethylester versetzt. Anschließend wurden bei 15 - 20°C 35,2 g (0,22 Mol) Brom zugetropft und 3 Stunden bei Raumtemperatur nachgerührt. Danach wurde der Niederschlag abgesaugt, mit Wasser nachgewaschen und getrocknet. Man erhielt so 19,9 g (70 % der Theorie) 4,5-Dibromimidazol-2-carbonsäuremethylester. Schmelzpunkt 211°C (aus wenig Acetonitril bzw. aus Toluol).

Herstellung des Ausgangsproduktes:

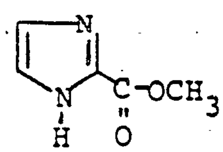

Eine Mischung aus 1,5 Liter Methanol und 141 g (0,75 Mol) des dimeren Ketens der Formel

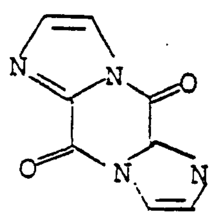

wurde eine halbe Stunde unter Rückfluß gerührt. Anschließend wurde heiß von geringfügigen Anteilen Ungelöstem abfiltriert und das Filtrat im Vakuum zur Trockne eingeengt. Man erhielt so den Imidazol-2-carbonsäuremethylester in praktisch quantitativer Ausbeute. Seine physikalischen Eigenschaften stimmen mit den in Helw. Chim. Acta 1976, 59, Seite 2738 genannten überein.

Beispiel 5

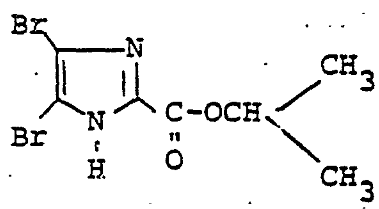

Nach der im Beispiel 4 angegebenen Methode wurde aus Imidazol-2-carbonsäure-isopropylester der 4,5-Dibrom-imidazol-2-carbonsäure-isopropylester vom Schmelzpunkt 205°C (aus Acetonitril) gewonnen.

Herstellung des Ausgangsproduktes:

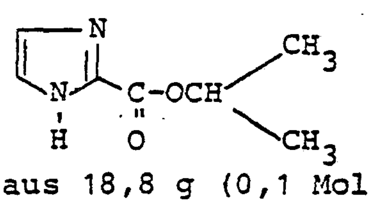

Eine Mischung aus 18,8 g (0,1 Mol) des dimeren Ketens der Formel

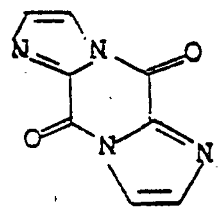

250 ml iso-Propanol und ca. 1 g pulverisiertem Natriumhydroxid wurde 4 Stunden unter Rückfluß gerührt. Anschließend wurde heiß von geringfügigen Anteilen Ungelöstem abfiltriert und das Filtrat im Vakuum zur Trockne eingeengt. Man erhielt so den Imidazol-2-carbonsäure-isoproyplester in praktisch quantitativer Ausbeute. Schmelzpunkt 166°C (aus Acetonitril).

Beispiel 6

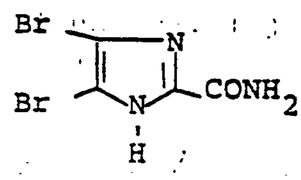

Zu einer Suspension von 11,1 g (0,1 Mol) Imidazol-2-carbonsäureamid in 110 ml Wasser wurden bei 5-10°C 35,2 g (0,22 Mol) Brom getropft und 2 Stunden bei Raumtemperatur nachgerührt. Anschließend wurde mit 1n-Natronlauge auf pH 6-7 eingestellt. Der Niederschlag wurde abgesaugt, mit Wasser nachgewaschen und getrocknet. Man erhielt so 16,8 g (62 % der Theorie) 4,5-Dibromimidazol-2-carbonsäureamid. Schmelzpunkt ca. 290°C (aus sehr wenig 1,4-Dioxan).

Beispiel 7

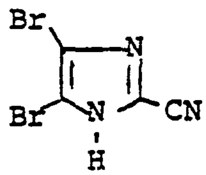

In ähnlicher Weise wie in Beispiel 6 beschrieben wurde aus Imidazol-2-carbonsäurenitril das 4,5-Dibromimidazol-2-carbonsäurenitril in 62,6 %iger Ausbeute gewonnen. Schmelzpunkt 228°C (aus Toluol). Die Verbindung läßt sich bei 180°C/0,01 Torr unzersetzt sublimieren.

Beispiel 8

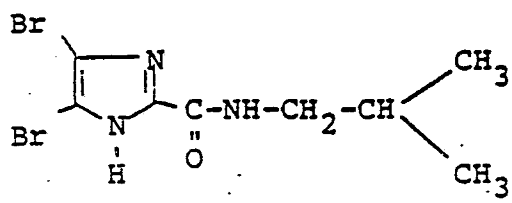

Nach der im Beispiel 6 beschriebenen Methode wurde aus Imidazol-2-carbonsäureisobutylamid das 4,5-Dibromimidazol-2-carbonsäureisobutylamid vom Schmelzpunkt 172°C (aus Acetonitril) gewonnen.

Die Herstellung des als Ausgangsprodukt benötigten Imidazol-2-carbonsäure-isobutylamids erfolgte aus Imidazol und iso-Butylisocyanat analg der in J. Org. Chem. 1977, 42, Seite 3925 für das Imidazol-2-carbonsäure -n-butylamid gegeben Vorschrift; Schmelzpunkt 202°C.

Beispiel 9

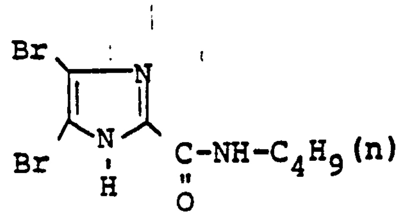

In ähnlicher Weise wie in Beispiel 6 beschrieben wurde aus Imidazol-2-carbonsäure-n-butylamid das 4,5-Dibromimidazol-2-carbonsäure-n-butylamid vom Schmelzpunkt 138°C (aus Cyclohexan) gewonnen.

Die Herstellung des als Ausgangsprodukt benötigten Imidazol-2-carbonsäure-n-butylamids erfolgte aus Imidazol und n-Butylisocyanat nach der in J. Org. Chem. 1977 (42), Seite 3925 beschriebenen Methode.

Beispiel 10

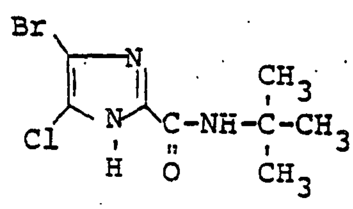

In einer Lösung von 6,45 g (0,026 Mol) 4-Brom-imidazol-2-carbonsäure-tert.-butylamid (Herstellung siehe Beispiel 2) in 100 ml Chloroform wurde unter Kühlung im Temperaturbereich von -10 bis 0°C eine Mischung aus 3,50 g (0,026 Mol) Sulfurylchlorid und

100 ml Chloroform innerhalb einer Stunde getropft. Nach etwa dreistündigem Nachrühren bei Raumtemperatur wurde das Chloroform im Vakuum abgezogen, der Rückstand mit 100 ml Salzsäure (1:1) verrührt, abgesaugt, mit Salzsäure (1:1) und Wasser nachgewaschen und getrocknet. Man erhielt so 5,10 g (70 % der Theorie) 4-Brom-5-chlor-imidazol-2-carbonsäure-tert.-butylamid vom Schmelzpunkt 226°C.

Beispiel 11

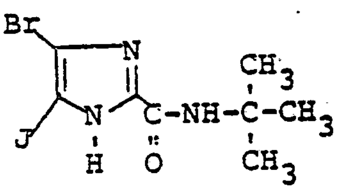

Eine Lösung von 6,33 g (0,0258 Mol) 4-Brom-imidazol-2-carbonsäure-tert.-butylamid (Herstellung siehe Beispiel 2) in 150 ml Chloroform wurde zunächst bei 20°C mit einer Lösung von 7,86 g (0,031 Mol) Jod in 200 ml Chloroform versetzt und anschließend 2 Stunden unter Rückfluß gerührt. Nach dem Abziehen des Chloroforms im Vakuum wurde der Rückstand mit 150 ml Salzsäure (1:1) verrührt, abgesaugt und mit Salzsäure (1:1) und Wasser nachgewaschen. Anschließend wurde der Rückstand in 40 ml 10 %iger Natronlauge gelöst, von geringen Mengen Ungelöstem abfiltriert und das klare Filtrat mit Salzsäure schwach angesäuert. Der hierbei entstandene Niederschlag wurde abgesaugt, mit

Wasser gewaschen und getrocknet. Man erhielt so 4,0 g (41,7 % der Theorie) 4-Brom-5-jod-imidazol-2-carbonsäure-tert.-butylamid vom Schmelzpunkt 248°C.

Beispiel 12

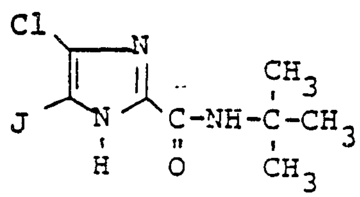

In ähnlicher Weise wie in Beispiel 11 beschrieben wurde aus 4-Chlor-imidazol-2-carbonsäure-tert.-butylamid (Herstellung siehe Beispiel 1) das 4-Chlor-5-jod-imidazol-2-carbonsäure-tert.-butylamid vom Schmelzpunkt 243°C gewonnen.

Beispiel 13

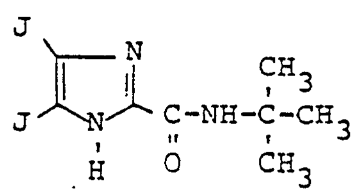

Zu einer Lösung von 16,7 g (0,1 Mol) Imidazol-2-carbonsäure-tert.-butylamid in 300 ml Methylenchlorid wurde bei Raumtemperatur eine Lösung von 51,8 g (0,204 Mol) Jod in 1000 ml Methylenchlorid getropft.

Nach einstündigem Nachrühren wurde das Methylenchlorid im Vakuum abgezogen, der Rückstand mit 250 g Salzsäure (1:1) verrührt, abgesaugt, mit Salzsäure (1:1) und Wasser nachgewaschen und anschließend in 10 %iger Natronlauge gelöst. Nach dem Abfiltrieren von geringen Mengen Ungelöstem wurde das Filtrat mit Salzsäure schwach angesäuert. Der hierbei entstandene Niederschlag wurde abgesaugt, mit Wasser gewaschen und getrocknet. Man erhielt so 29,8 g (71 % der Theorie) 4,5-Dijodimidazol-2-carbonsäure-tert.-butylamid. Die Verbindung ist bei 290°C noch nicht geschmolzen. Sie ist bei 180°C/0,01 Torr unzersetzt in farblosen Kristallen sublimierbar und läßt sich aus Toluol umkristallisieren.

Beispiel 14

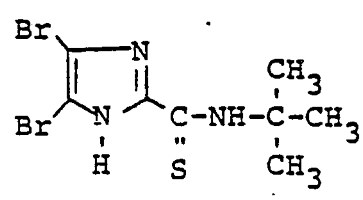

50 g (0,15 Mol) 4,5-Dibrom-imidazol-2-carbonsäure-tert.-butylamid (Herstellung siehe Beispiel 3) und 50 g Phosphorpentasulfid ($P_4S_{10}$) wurden in 750 ml Xylol 5 Stunden unter Rückfluß gerührt. Nach dem Abziehen des Xylols im Vakuum wurde der Rückstand mit etwa 1 Liter gesättigter wäßriger Natriumhydrogencarbonat-Lösung allmählich bis zum Sieden erwärmt.

Nach Filtration in der Siedehitze wurde das Filtrat abgekühlt und mit verdünnter Salzsäure angesäuert. Der hierbei entstandene Niederschlag wurde abgesaugt, mit Wasser gewaschen und getrocknet. Nach Extraktion mit siedendem Petroläter (Siedebereich 40-80°C) und langsamem Abkühlen erhielt man hellgelbe bis honiggelbe, derbe Kristalle vom Schmelzpunkt 177°C.

Analog Beispiel 6 wurden die folgenden 4,5-Dibromimidazol-2-carbonsäureamide der Formel

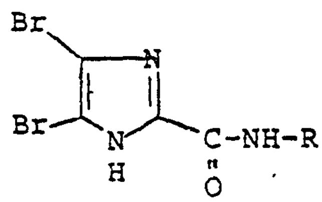

gewonnen:

Tabelle 1

| Beispiel | R | Schmelzpunkt (°C) | umkristallisiert aus |
|---|---|---|---|
| 15 | $CH_3$ | 270 | Acetonitril |
| 16 | $C_2H_5$ | 166 | Acetonitril |
| 17 | $n-C_3H_7$ | 171 | Acetonitril |
| 18 | $i-C_3H_7$ | 166 | Acetonitril |
| 19 | $-CH_2-C(CH_3)_3$ | 216 | Acetonitril |
| 20 | sek.-$C_4H_9$ | 146 | Acetonitril |

Die in der nachfolgenden Tabelle 2 formelmäßig aufgeführten Stoffe wurden nach der im Beispiel 3a angegebenen Methode hergestellt.

Tabelle 2

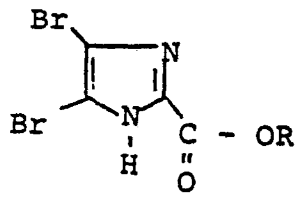

| Beispiel | R | Schmelzpunkt °C | umkristallisiert aus |
|---|---|---|---|
| 21 | $n-C_3-H_7$ | 96 | Cyclohexan |
| 22 | $n-C_4H_9$ | 112 | Cyclohexan |
| 23 | sek-$C_4H_9$ | 181 | Acetonitril |
| 24 | iso-$C_4H_9$ | 172 | Acetonitril |
| 25 | tert.-$C_4H_9$ | 190 (Zers.) | Acetonitril |
| 26 | $n-C_5H_{11}$ | 73 | Hexan |
| 27 | $-CH_2-CH_2-CH(CH_3)_2$ | 98 | Hexan |
| 28 | $-CH_2-C(CH_3)_3$ | 200 | Acetonitril |
| 29 | $n-C_6H_{13}$ | 72 | Hexan |
| 30 | $-\underset{CH_3}{CH}-CH_2-CH(CH_3)_2$ | 122 | Hexan |
| 31 | $-CH_2-CH(C_2H_5)_2$ | 110 | Cyclohexan |

Die Herstellung der als Ausgangsprodukte benötigten Imidazol-2-carbonsäureamide der Formel

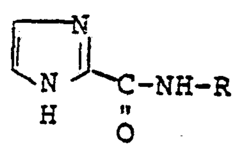

erfolgt ähnlich der in Beispiel 1 beschriebenen Herstellung des Imidazolyl-2-carbonsäure-tert.-butylamids.

Tabelle 3

| Beispiel-Nr. | R | Schmelzpunkt (°C) | umkristallisiert aus |
|---|---|---|---|
| 15 a | $CH_3$ | 260 | Wasser |
| 16 a | $C_2H_5$ | 210 | Wasser |
| 17 a | $n-C_3H_7$ | 207 | Acetonitril |
| 18 a | $i-C_3H_7$ | 226 | Acetonitril |
| 19 a | $-CH_2-C(CH_3)_3$ | 184 | Acetonitril |
| 20 a | sek.-$C_4H_9$ | 209 | Acetonitril |

Die in der nachfolgenden Tabelle 4 formelmäßig aufgeführten Ausgangsprodukte wurden nach der im Beispiel 5 angegebenen Methode hergestellt.

Tabelle 4

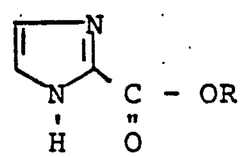

| Beispiel-Nr. | R | Schmelzpunkt in °C | umkristallisiert aus |
|---|---|---|---|
| 21 a | $n-C_3H_7$ | 129 | Acetonitril |
| 22 a | $n-C_4H_9$ | 134 | " |
| 23 a | sek.-$C_4H_9$ | 182 | " |
| 24 a | iso-$C_4H_9$ | 153 | " |
| 25 a | tert.-$C_4H_9$ | 170 | " |
| 26 a | $n-C_5H_{11}$ | 138 | " |
| 27 a | $-CH_2-CH_2-CH(CH_3)_2$ | 130 | " |
| 28 a | $-CH_2-C(CH_3)_3$ | 150 | " |

Tabelle 4 (Fortsetzung)

| Beispiel Nr. | R | Schmelzpunkt in °C | umkristallisiert aus |
|---|---|---|---|
| 29 a | $n-C_6H_{13}$ | 124 | Acetonitril |
| 30 a | $-\underset{CH_3}{CH}-CH_2-CH(CH_3)_2$ | 152 | " |
| 31 a | $-CH_2-CH(C_2H_5)_2$ | 132 | " |

Die gute herbizide Wirksamkeit der erfindungsgemäßen Stoffe geht aus den folgenden Verwendungsbeispielen hervor.

In diesen Beispielen wurde die nachstehend formelmäßig angegebene Substanz als Vergleichspräparat eingesetzt:

A = 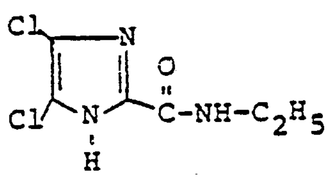 (bekannt)

(4,5-Dichlorimidazol-2-carbonsäure-ethylamid)

Beispiel A

Pre-emergence-Test

Lösungsmittel: 5 Gewichtsteile Aceton
Emulgator: 1 Gewichtsteil Alkylarylpolyglcoläther

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubreitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

In diesem Test zeigen die erfindungsgemäßen Wirkstoffe (3), (10), (11), (12) und (14) eine bessere Wirksamkeit als die Vergleichssubstanz (A).

Beispiel B

Post-emergence-Test

Lösungsmittel: 5 Gewichtsteile Aceton
Emulgator: 1 Gewichtsteil Alkylarylpolyglycoläther

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschte Wirkstoffmenge pro Flächeneinheit ausgebracht wird. Die Konzentration der Spritzbrühe wird so gewählt, daß in 2000 l Wasser/ha die jeweils gewünschte Wirkstoffmenge ausgebracht wird. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

In diesem Test zeigen die erfindungsgemäßen Verbindungen (3), (5), (10) und (13) eine bessere Wirksamkeit als die Vergleichssubstanz (A).

Patenansprüche

1) Halogenierte Imidazol-carbonsäure-Derivate der Formel

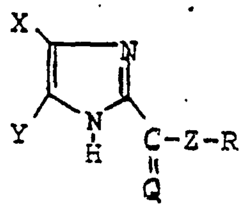

(I)

in welcher

X für Chlor, Brom oder Jod steht,

Y für Wasserstoff, Chlor, Brom oder Jod steht, wobei jedoch X und Y nicht gleichzeitig für Chlor stehen,

Z für Sauerstoff oder für die NH-Gruppe steht,

R für Wasserstoff oder Alkyl steht und

Q für Sauerstoff oder Schwefel steht oder der Rest der Formel $-\underset{\underset{Q}{\|}}{C}-Z-R$ für $-C{\equiv}N$ steht.

2) Halogenierte Imidazol-carbonsäure-Derivate der Formel (I), in denen R für geradkettiges oder verzweigtes Alkyl mit 1 bis 18 Kohlenstoffatomen steht.

3) Verfahren zur Herstellung von halogenierten Imidazol-carbonsäure-Derivaten der Formel

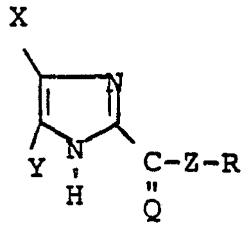

(I)

in welcher

X für Chlor, Brom oder Jod steht,

Y für Wasserstoff, Chlor, Brom oder Jod steht, wobei jedoch X und Y gleichzeitig für Chlor stehen,

Z für Sauerstoff oder für die NH-Gruppe steht,

R für Wasserstoff oder Alkyl steht und

Q für Sauerstoff oder Schwefel steht oder der Rest der Formel $-\underset{\underset{Q}{\|}}{C}-Z-R$ für $-C\equiv N$ steht,

dadurch gekennzeichnet, daß man

a) Imidazol-2-carbonsäure-Derivate der Formel

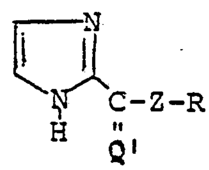

(II)

in welcher

Z und R die oben angegebene Bedeutung haben und

Q' für Sauerstoff steht oder der Rest der Formel $-\underset{\underset{Q'}{\|}}{C}-Z-R$ für -CN steht,

mit maximal der zur Monohalogenierung stöchiometrisch erforderlichen Menge an Halogenierungsmittel in Gegenwart eines unter den Reaktionsbedingungen inerten Verdünnungsmittels umsetzt,

oder

b) Imidazol-2-carbonsäure-Derivate der Formel

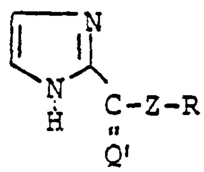

(II)

in welcher

Z, R und Q' die oben angegebene Bedeutung haben,

mit mindestens der zur Dihalogenierung stöchiometrisch erforderlichen Menge an Halogenierungsmittel in Gegenwart eines unter den Reaktionsbedingungen inerten Verdünnungsmittels sowie gegebenenfalls in Gegenwart eines säurebindenden Mittels umsetzt,

oder

c) monohalogenierte Imidazol-2-carbonsäure-Derivate der Formel

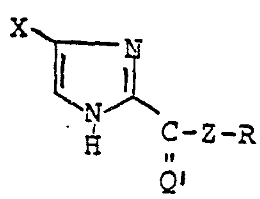

(III)

in welcher

X, Z, R und Q' die oben angegebene Bedeutung haben,

mit der stöchiometrisch erforderlichen Menge oder mit einem Überschuß an Halogenierungsmittel in Gegenwart eines unter den Reaktionsbedingungen inerten Verdünnungsmittels sowie gegebenenfalls in Gegenwart eines Säurefängers umsetzt,

oder

d) halogenierte Imidazol-carbonsäure-Derivate der Formel

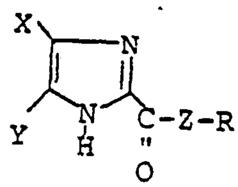

(IV)

in welcher

X, Y, Z und R die oben angebene Bedeutung haben,

mit mindestens der stöchiometrisch erforderlichen Menge an Tetraphosphor-dekasulfid ("Phosphorpentasulfid") in Gegenwart eines unter den Reaktionsbedingungen inerten Verdünnungsmittels umsetzt.

4) Herbizide Mittel, gekennzeichnet, durch einen Gehalt an mindestens einem halogenierten Imidazol-carbonsäure-Derivat der Formel (I) gemäß Anspruch 1.

5) Verfahren zur Bekämpfung von Unkräutern, dadurch gekennzeichnet, daß man halogenierte Imidazol-carbonsäure-Derivate der Formel (I) gemäß Anspruch 1 auf Unkräuter und/oder deren Lebensraum ausbringt.

6) Verwendung von halogenierten Imidazol-carbonsäure-Derivaten der Formel (I) gemäß Anspruch 1 zur Bekämpfung von Unkräutern.

7) Verfahren zur Herstellung von herbiziden Mitteln, dadurch gekennzeichnet, daß man halogenierte Imidazol-carbonsäure-Derivate der Formel (I) gemäß Anspruch 1 mit Streckmitteln und/oder oberflächenaktiven Stoffen vermischt.

8) Halogeniertes Imidazol-carbonsäure-Derivat der Formel

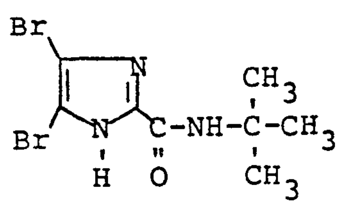

9) Halogeniertes Imidazol-carbonsäure-Derivat der Formel

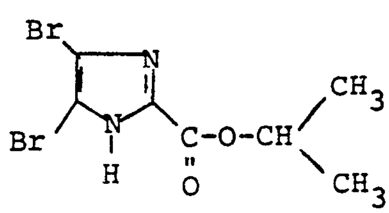

10) Halogeniertes Imidazol-carbonsäure-Derivat der Formel

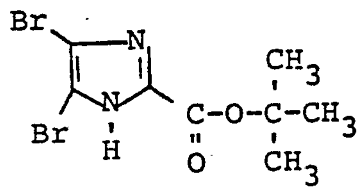

0031086

Europäisches Patentamt

# EUROPÄISCHER RECHERCHENBERICHT

Nummer der Anmeldung
EP 80 10 7805

| EINSCHLÄGIGE DOKUMENTE | | | KLASSIFIKATION DER ANMELDUNG (Int. Cl.³) |
|---|---|---|---|
| Kategorie | Kennzeichnung des Dokuments mit Angabe, soweit erforderlich, der maßgeblichen Teile | betrifft Anspruch | |
| X | DE - A - 1 567 084 (SHELL)<br>* Seiten 1-3 *<br>--- | 1,4-7 | C 07 D 233/90<br>A 01 N 43/50 |
| D,A | DE - A - 2 610 527 (BAYER)<br>* Seiten 1-12 *<br>--- | 1,4-7 | |
| P | JOURNAL OF HETEROCYCLIC CHEMISTRY, Band 17, Nr. 2, März 1980, UTAH (US)<br>J.P. DIRLAM et al.: "Syntheses and Reactions of Some 4,5-Dihaloimidazole-2-carboxylic Acid Derivatives", Seiten 409-411<br>* Seiten 409-411 *<br>--------- | 1,3 | RECHERCHIERTE SACHGEBIETE (Int Cl.³)<br>C 07 D 233/90 |

KATEGORIE DER GENANNTEN DOKUMENTE
X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 11.03.1981 | DE BUYSER |

EPA form 1503.1 06.78